# EUROPÄISCHE PATENTSCHRIFT

(11) **EP 1 781 263 B1**
(45) Veröffentlichungstag und Bekanntmachung des Hinweises auf die Patenterteilung: **01.10.2008**
(21) Anmeldenummer: 05746448.9
(22) Anmeldetag: 18.05.2005
(51) Int. Cl.: A61K 9/28

(54) **MEHRSCHICHTIGE ARZNEIFORM**
MEDICAMENT IN A MULTILAYER FORM
MEDICAMENT SOUS FORME MULTICOUCHE

(30) Priorität: 23.07.2004 DE 102004035936
(43) Veröffentlichungstag der Anmeldung: 09.05.2007
(73) Patentinhaber: Evonik Röhm GmbH, 64293 Darmstadt (DE)
(72) Erfinder: PETEREIT, Hans-Ulrich, 64291 Darmstadt (DE); MEIER, Christian, 64295 Darmstadt (DE)
(86) Internationale Anmeldenummer: PCT/EP2005/005374
(87) Internationale Veröffentlichungsnummer: WO 2006/010394

(56) Entgegenhaltungen:
- EP-A- 0 519 870
- EP-A- 0 667 148
- WO-A-01/68058
- WO-A-97/02020
- WO-A-20/04039357

## Beschreibung

Die Erfindung betrifft eine mehrschichtige Arzneiform, aufgebaut aus einem Kern mit einem pharmazeutischen Wirkstoff, einem inneren Polymerüberzug und einem äußeren Polymerüberzug.

### Stand der Technik

EP 0 704 207 A2 beschreibt thermoplastische Kunststoffe für darmsaftlösliche Arzneiumhüllungen. Es handelt sich dabei um Mischpolymerisate aus 16 bis 40 Gew.-% Acryl- oder Methacrylsäure, 30 bis 80 Gew.-% Methylacrylat und 0 bis 40 Gew.-% anderen Alkylestern der Acrylsäure und/oder Methacrylsäure.

EP 0 704 208 A2 beschreibt Überzugs- und Bindemittel für darmsaftlösliche Arzneiumhüllungen. Es handelt sich dabei um Copolymerisate aus 10 bis 25 Gew.-% Methacrylsäure, 40 bis 70 Gew.-% Methylacrylat und 20 bis 40 Gew-% Methylmethacrylat. Die Beschreibung erwähnt neben einschichtigen Überzügen auch mehrlagige Überzugssysteme. Diese können aus einem Kern, der z. B. einen basischen oder einen wasserempfindlichen Wirkstoff enthält, bestehen, weisen eine Isolierschicht aus einem anderen Überzugsmaterial, wie Celluloseether, Celluloseester oder einem kationischen Polymethacrylat z. B. von Typ EUDRAGIT® E, RS oder RL auf und werden dann zusätzlich mit der oben genannten darmsaftlöslichen Umhüllung versehen.

EP 0 519 870 A1 beschreibt orale Diclofenac-Zubereitungen. Der Wirkstoff ist dabei auf einem Kern aufgetragen, der zweischichtig überzogen ist. Die innere Schicht kann aus einem neutralen (Meth)acrylatcopolymeren von Typ EUDRAGIT^{®} NE bestehen und enthält neben den pharmazeutisch üblichen Hilfsstoffen, wie z. B. Trennmitteln, 5 bis 20 Gew.-% eines Porenbildners, z. B. Eisenoxid rot. Die äußere Schicht ist magensaftresistent und kann z. B. aus einem (Meth)acrylatcopolymer von Typ EUDRAGIT^{®} L bestehen.

US 5,643,602 beschreibt orale pharmazeutische Arzneiformen zur Therapie der Colitis ulcerosa oder Morbus Crohn. Die Arzneiform hat einen mehrschichtigen Aufbau mit einem neutraler Kern in Inneren und darauf folgend zwei Polymerschichten. Der Wirkstoff liegt dabe in einer inneren Schicht in Mischung mit einem neutralen Polymeren, z. B. Ethycellulose oder EUDRAGIT^{®} NE vor. Die äußere Schicht ist magensaftresistent und kann z. B. aus einem (Meth)acrylatcopolymer von Typ EUDRAGIT^{®} L bestehen.

WO 01/68058 beschreibt eine mehrschichtige Arzneiform, die im wesentlichen aufgebaut ist aus a) einem Kern mit einem pharmazeutischen Wirkstoff, b) einem inneren Überzug aus einem Copolymeren oder einer Mischung von Copolymeren, die sich aus 85 bis 98 Gew.-% radikalisch polymerisierten C1-bis C4-Alkylestern der Acryl- oder der Methacrylsäure und 15 bis 2 Gew.-% (Meth)acrylat-Monomeren mit einer quaternären Ammoniumgruppe im Alkylrest zusammensetzen und c) einem äußeren Überzug aus einem Copolymeren, das sich aus 75 bis 95 Gew.-% radikalisch polymerisierten C₁- bis C₄-Alkylestern der Acryl- oder der Methacrylsäure und 5 bis 25 Gew.-% (Meth)acrylatMonomeren mit einer anionischen Gruppe im Alkylrest zusammensetzt.

WO 2004/039357 beschreibt eine mehrschichtige Arzneiform, aufgebaut aus a) einem neutralen Kern, b) einem inneren Überzug aus einem Methacrylat-Copolymeren und c) einem äußeren Überzug aus einem Copolymeren, das sich aus 40 bis 95 Gew.-% radikalisch polymerisierten C₁- bis C₄-Alkylestern der Acryl- oder der Methacrylsäure und 5 bis 60 Gew.-% (Meth)acrylatMonomeren mit einer anionischen Gruppe im Alkylrest zusammensetzt. Die Arzneiform ist dadurch gekennzeichnet, daß der innere Überzug im wesentlichen aus einem Methacrylat-Copolymeren besteht, das mindestens zu 90 Gew.-% aus (Meth)acrylat-Monomeren mit neutralen Resten aufgebaut ist, eine Mindestfilmbildetemperatur nach DIN 53 787 von höchstens 30°C aufweist und den pharmazeutischen Wirkstoff in gebundener Form enthält.

### Aufgabe und Lösung

Arzneiformen gemäß der WO 01/68058 weist hervorragende Eigenschaften für Freisetzung von Wirkstoffen im Colon auf. Im Magen wird nahezu kein Wirkstoff abgegeben und es wird eine gleichmäßige und langanhaltende Wirkstoffabgabe im Darm, insbesondere kurz vor oder erst im Dickdarmbereich erreicht. Die Art der Wirkstoffabgabe ist dergestalt, daß die in-vitro Anforderung erfüllt wird, daß im Freisetzungstest nach USP zwei Stunden bei pH 1,2 und anschließendem Umpuffern auf pH 7,0 der enthaltene Wirkstoff im Zeitraum bis 2,0 Stunden nach Testbeginn zu weniger als 5 % und zum Zeitpunkt acht Stunden nach Testbeginn zu 30 bis 80 % freigesetzt wird.

Es wurde jedoch festgestellt, dass die Überzüge der beschriebenen Arzneiform nicht immer geeignete mechanische Eigenschaften aufweist. Insbesondere bei sehr dünnen Filmhüllen, z. B. bei schwerlöslichen oder hochdosierten Arzneistoffen besteht der Bedarf erhöhter mechanischer Festigkeit zur Stabilisierung der Filmüberzüge bei pharmaüblichen Produktionsvorgängen wie Verpressen, Abfüllen in Kapseln bzw. Sachets oder Mischen mit anderen Pelletpräparationen. Ähnliches gilt für Arzneiformen gemäß der EP 0 519 870 A1 oder WO 2004/039357.

Es wurde somit als eine Aufgabe gesehen, eine Arzneiform mit zumindest sehr ähnlicher Freisetzungscharakteristik bereitzustellen, die jedoch in den mechanischen Eigenschaften des Filmüberzugs verbessert ist.

Die Aufgabe wird gelöst durch eine mehrschichtige Arzneiform, enthaltend
a) einen Kern mit einem pharmazeutischen Wirkstoff
b) einen inneren Überzug, der zu 50 bis 95 Gew.-% aus einem (Co)polymeren besteht, das sich zu 95 bis 100 Gew.-% aus radikalisch polymerisierten vinylischen Monomeren mit neutralen Seitengruppen und 0 bis 5 Gew.-% Monomeren mit anionischen Seitengruppen zusammensetzt,
c) einen äußeren Überzug aus einem Copolymeren, das sich aus 75 bis 95 Gew.-% radikalisch polymerisierten C₁- bis C₄-Alkylestern der Acryl- oder der Methacrylsäure und 5 bis 25 Gew.-% (Meth)acrylat-Monomeren mit einer anionischen Gruppe im Alkylrest zusammensetzt, wobei 5 bis 30 Gew.-% an pharmazeutisch üblichen Hilfsstoffen, insbesondere Weichmacher, enthalten sind,
dadurch gekennzeichnet, daß
der innere Überzug 5 bis 50 Gew.-% an pharmazeutisch üblichen Hilfsstoffen enthält, die keine Porenbildner sind und Porenbildner nur in Mengen von weniger als 5 Gew.-% enthalten sind.

Die Kombination des inneren und der äußeren Überzugsfilms führen offenbar in zu einer gesteigerten Zugfestigkeit der doppelten Filmschicht als Ganzes im Vergleich zu WO 01/68058. Dadurch werden die mechanischen Eigenschaften der Arzneiform selbst und daraus hergestellter mulipartikulärer Arzneiformen deutlich verbessert. Man kann die Eigenschaftsverbesserung an isolierten doppelten Filmschichten erkennen. Bei erfindungsgemäß aufgebauten, isolierten Doppelfilmschichten werden Zugfestigkeiten im Bereich von 6 bis 10 [Mpa] und nominale Bruchdehnungen im Bereich von 170 bis 300 [%] gemessen.

### Ausführung der Erfindung

### Die Erfindung betrifft eine mehrschichtige Arzneiform, enthaltend

### Kern a)

Träger bzw. Kerne für die Überzüge sind Tabletten, Granulate, Pellets, Kristalle von regelmäßiger oder unregelmäßiger Form. Die Größe von Granulaten, Pellets oder Kristallen liegt in der Regel zwischen 0,01 und 2,5 mm, die von Tabletten zwischen 2,5 und 30,0 mm. Die Träger enthalten üblicherweise zu 1 bis 95 % Wirkstoff sowie gegebenenfalls bzw. in der Regel weitere pharmazeutische Hilfsstoffe.

Übliche Herstellungsverfahren sind direktes Verpressen, Verpressen von Trocken-, Feucht- oder Sintergranulaten, Extrusion und anschließende Ausrundung, feuchte oder trockene Granulation oder direkte Pelletierung (z.B. auf Tellern) oder durch Binden von Pulvern (Powder layering) auf wirkstofffreie Kugeln (Nonpareilles) oder wirkstoffhaltige Partikeln.

Neben dem Wirkstoff können die Kerne weitere pharmazeutische Hilfsstoffe enthalten: Bindemittel, wie Lactose, Cellulose und deren Derivate, Polyvinylpyrrolidon (PVP), Feuchthaltemittel, Zerfallsförderer, Gleitmittel, Sprengmittel, Stärke und deren Derivate, Zucker Solubilisatoren oder andere.

Die Kerne a) können in üblicher Weise mit einem pharmazeutischen Wirkstoff versehen werden, indem man den entsprechenden Wirkstoff, z. B. als Wirkstoffpulver auf Trägerpartikel (Nonpareilles) mittels eines wäßrigen Bindemittels aufbringt. Die Wirkstoffkerne (Pellets) können nach Trocknung und Siebung in der gewünschten Größenfraktion erhalten werden (z. B. 0,7 bis 1 mm). Man bezeichnet dieses Verfahren u.a. als "Powder Layering". Der Wirkstoffgehalt des Kerns kann z. B. 5 bis 90 Gew.-% betragen.

### Innerer Überzug b)

Der innere Überzug b) besteht zu 50 bis 95, bevorzugt zu 60 bis 90 Gew.-% aus einem (Co)polymeren, das sich zu 95 bis 100, bevorzugt zu 98 bis 100 Gew.-% aus radikalisch polymerisierten vinylischen Monomeren mit neutralen Seitengruppen und 0 bis 5, bevorzugt 0 bis 2 Gew.-% vinylischen Monomeren mit anionischen Seitengruppen zusammensetzt. Das überwiegend oder völlig neutrale Copolymer hat bevorzugt die Eigenschaft, oberhalb von pH 5,0 in Wasser bzw. im Darmsaftmillieu zu quellen und den Wirkstoff kontrolliert bzw. retardiert freizusetzen.

Die Wirkstofffreisetzungscharakteristik entspricht nicht exakt derjenigen, wie sie in der WO 01/68058 beschrieben ist, die Abweichungen sind jedoch überraschend gering. Die Modifikation zugunsten der besseren mechanischen Eigenschaften erscheint deshalb durchgängig tolerierbar. Durch Variation der Schichtdicke des inneren Überzuges kann das Freigabeprofil gegebenenfalls angeglichen werden.

Der innere Überzug kann ein (Co)polymer enthalten, das sich aus 95 bis 100, bevorzugt 98 bis 100 Gew.-% radikalisch polymerisierten C₁- bis C₄-Alkylestern der Acryl- oder der Methacrylsäure und gegebenenfalls 0 bis 5, bevorzugt 0 bis 2 Gew.-% vinylischen Monomeren mit anionischen Seitengruppen, insbesondere Acryl- und/oder Methacrylsäure, zusammensetzt.

C₁- bis C₄-Alkylestern der Acryl- oder Methacrylsäure sind insbesondere Methylmethacrylat, Ethylmethacrylat, Butylmethacrylat, Methylacrylat, Ethylacrylat und Butylacrylat.

Ein (Meth)acrylat-Monomer mit einer anionischen Gruppe im Alkylrest kann z. B. Acrylsäure, bevorzugt jedoch Methacrylsäure sein.

Geeignet sind z. B. neutrale (Meth)acrylat Copolymere aus 20 bis 40 Gew.-% Ethylacrylat und 60 bis 80 Gew.-% Methylmethacrylat (Typ EUDRAGIT^{®} NE).

EUDRAGIT^{®} NE ist ein Copolymer aus 30 Gew.-% Ethylacrylat und 70 Gew.-% Methylmethacrylat.

Der innere Überzug kann ein (Co)polymer enthalten, das Polyvinylacetat bzw. ein Polyvinylacetat ist. Der Begriff "ein Polyvinylacetat" schließt Derivate des Polyvinylacetats mit ein. Das Polyvinylacetat kann als Dispersion vorliegen (z. B. vom Typ Kollicoat^{®} SR 30 D, Hersteller BASF, Polyvinylacetat Dispersion, stabilisiert mit Povidon und Na-Laurylsulfat).

Der innere Überzug enthält 5 bis 50 Gew.-% an pharmazeutisch üblichen Hilfsstoffen, die keine Porenbildner sind.

Es wurde festgestellt, daß Porenbildner, wie sie in der EP 0 519 870 A1 verwendet werden, die mechanischen Eigenschaften der doppelten Überzugsfilmschicht nachteilig beeinflussen, wenn diese, wie in der EP 0 519 870 A1, in der inneren Schicht vorhanden sind. Die innere Überzugsschicht kann, auch wenn dies nicht zweckmäßig erscheint, in geringer Menge Porenbildner enthalten, ohne, dass die mechanischen Eigenschaften des doppelten Überzugs zwangsläufig zu sehr beeinträchtigt werden. Porenbildner sollten im inneren Überzug bevorzugt nicht oder nur in Mengen von weniger als 5, bevorzugt weniger als 2 oder 1 Gew.-% verwendet werden. Derartig geringe Mengen bewirken in der Regel keinen technischen Effekt mehr. Besonders bevorzugt sind deshalb keine Porenbildner in der inneren Überzugsschicht enthalten.

Die pharmazeutisch üblichen Hilfsstoffe, die im inneren Überzug enthalten sein können, werden aus den Stoffklassen der Weichmacher, Stabilisatoren, Farbstoffe, Antioxidantien, Netzmittel, Pigmente, Glanzmittel, Trennmittel, Trockenstellmittel ausgewählt, wobei Porenbildner, insbesondere wasserunlösliche Porenbildner wie Kaolin, Calciumcarbonat, Calciumhydrogenphosphat, Magnesiumoxid, mikrokristalline Cellulose, Titandioxid oder Eisenoxid, und insbesondere wasserlösliche Porenbildner wie Povidone K30, Polyvinylalkohol, Cellulosederivate, wie Hydroxypropylcellulose, Hydroxypropylmethylcellulose (HPMC), Methylcellulose oder Natriumcarboxymethylcellulose, Saccharose, Xylit, Sorbit, Mannit, Maltose, Xylose, Glucose, Kaliumchlorid, Natriumchlorid, Polysorbat 80, Poylethylenglykol oder Natriumcitrat nicht oder nur in Mengen von weniger als 5, bevorzugt weniger als 2 oder 1 Gew.-% enthalten sind.

Es wurde weiterhin festgestellt, daß ein in der inneren Überzugsschicht gebundener Wirkstoff, wie in der WO 2004/039357 angeregt, die mechanischen Eigenschaften der doppelten Überzugsfilmschicht ebenfalls nachteilig beeinflusst. Der in der Arzneiform enthaltene Wirkstoff wird zweckmäßigerweise in der Kernschicht untergebracht. Die innere Überzugsschicht kann, auch wenn dies nicht zweckmäßig erscheint, in geringer Menge etwas Wirkstoff enthalten, ohne, daß die mechanischen Eigenschaften des Überzugs zwangsläufig zu sehr beeinträchtigt werden. Der Wirkstoffgehalt sollte im inneren Überzug jedoch bei weniger als 2, bevorzugt weniger als 1 liegen. Derartig geringe Mengen bewirken in der Regel keinen technischen Effekt mehr. Besonders bevorzugt ist deshalb kein Wirkstoff in der inneren Überzugsschicht enthalten.

Die Schichtdicke des inneren Überzugs kann z. B. im Bereich von 10-100, bevorzugt 20 bis 40 µm liegen.

### Äußerer Überzug c)

Der äußere Überzug c) enthält ein Copolymer, das sich aus 75 bis 95 Gew.-% radikalisch polymerisierten C₁- bis C₄-Alkylestern der Acryl- oder der Methacrylsäure und 5 bis 25 Gew.-% (Meth)acrylat-Monomeren mit einer anionischen Gruppe im Alkylrest zusammensetzt, wobei 5 bis 30, bevorzugt 8 bis 20 Gew.-% an pharmazeutisch üblichen Hilfsstoffen, insbesondere Weichmacher, enthalten sind. Porenbildner sollen im äußeren Überzug bevorzugt nicht oder nur in Mengen von weniger als 5, bevorzugt weniger als 2 oder 1 Gew.-% verwendet werden. Derartig geringe Mengen bewirken in der Regel keinen technischen Effekt mehr. Besonders bevorzugt sind deshalb keine Porenbildner in der äußeren Überzugsschicht enthalten.

C₁- bis C₄-Alkylestern der Acryl- oder Methacrylsäure sind insbesondere Methylmethacrylat, Ethylmethacrylat, Butylmethacrylat, Methylacrylat, Ethylacrylat und Butylacrylat.

Ein (Meth)acrylat-Monomer mit einer anionischen Gruppe im Alkylrest kann z. B. Acrylsäure, bevorzugt jedoch Methacrylsäure sein.

Besonders gut geeignet sind (Meth)acrylat Copolymere aus 10 bis 30 Gew.-%, Methylmethacrylat, 50 bis 70 Gew.-% Methylacrylat und 5 bis 15 Gew.-% Methacrylsäure (Typ EUDRAGIT^{®} FS).

Die Copolymere sind handelsüblich und können in an sich bekannter Weise durch radikalische Substanz-, Lösungs-, Perl- oder Emulsionspolymerisation erhalten werden. Sie müssen vor der Verarbeitung durch geeignete Mahl-, Trocken- oder Sprühprozesse in den erfindungsgemäßen Teilchengrößenbereich gebracht werden. Dies kann durch einfaches Brechen extrudierter und abgekühlter Granulatstränge oder Heißabschlag erfolgen.

Bevorzugt ist die Emulsionspolymerisation in wäßriger Phase in Gegenwart wasserlöslicher Initiatoren und (vorzugsweise anionischer) Emulgatoren (Siehe z. B. DE-C 2 135 073).

Das Emulsionspolymerisat wird vorzugsweise in Form einer 10- bis 50-gew.-prozentigen, insbesondere 30 bis 40-prozentigen wäßrigen Dispersion erzeugt und angewendet. Für die Verarbeitung ist eine teilweise Neutralisation der Methacrylsäure-Einheiten entbehrlich; sie ist jedoch, beispielsweise in einem Umfang bis zu 5 oder 10 Mol-% möglich, wenn eine Verdickung der Überzugsmitteldispersion erwünscht sein sollte. Der Gewichtsmittelwert der Latex-Teilchengröße beträgt in der Regel 40 bis 100 nm, vorzugsweise 50 bis 70 nm, was eine verarbeitungstechnisch günstige Viskosität unter 1000 mPa · s gewährleistet.

Die Schichtdicke des äußeren Überzugs kann z. B. im Bereich von 20 - 150, bevorzugt 40 bis 80 µm liegen.

### Mengenverhältnisse innerer/äußerer Überzug

Das Gesamtgewicht des inneren Überzugs kann bevorzugt 2 bis 50, besonders bevorzugt 10 bis 40 Gew.-% bezogen auf das Gesamtgewicht des Kerns ausmachen.

Das Gesamtgewicht des Kerns setzt sich aus dem Wirkstoff, den gegebenenfalls zur Formulierung verwendete Hilfsstoffen einschließlich gegebenenfalls verwendeter neutraler Kerne (Non-Pareilles) zusammen, entspricht also dem Trockengewicht der Formulierung.

Das Gesamtgewicht des inneren Überzugs setzt sich aus dem Copolymeren und den enthaltenen Hilfsstoffen zusammen, entspricht also dem Trockengewicht der verwendeten Formulierung.

Das Gesamtgewicht des äußeren Überzugs setzt sich aus dem Copolymeren und den gegebenenfalls enthaltenen Hilfsstoffen, z. B. Weichmacher, zusammen, entspricht also dem Trockengewicht der verwendeten Formulierung.

Das Gesamtgewicht des äußeren Überzugs kann bevorzugt 5 bis 50, besonders bevorzugt 10 bis 30 Gew.-% bezogen auf das Gesamtgewicht des Kerns und des inneren Überzugs ausmachen.

Im Übrigen zeigen rasterelekronenmikroskopische Aufnahmen von Querschnitten isolierter Doppelfilme mit erfindungsgemäßem Aufbau homogene, gleichmäßige Schichten mit guter Haftung an der Grenzfläche.

### Verfahren

Die Erfindung betrifft weiterhin ein Verfahren zur Herstellung der erfindungsgemäßen Arzneiform, gekennzeichnet durch die Schritte
a) Erzeugen eines Kerns mit einem pharmazeutischen mittels Sprühauftrag auf einen Neutral-Kern (Non-Pareilles) oder durch Rotagglomeration, Ausfällen, Sprühverfahren oder Extrusion und Spheronisation ohne einen Neutral-Kern herstellt und anschließend
b) Auftragen des inneren Überzugs, mittels Sprühauftrag, so daß wirkstoffhaltige, umhüllte Pellets erhalten werden,
c) Auftragen des äußeren Überzugs, mittels Sprühauftrag, so daß wirkstoffhaltige, zweifach umhüllte Pellets erhalten werden,
d) optional eine abschließende Curing-Behandlung zur Stabilisierung des Freigabeprofils, z. B. durch trockenes Lagern für 2 Stunden bei 40 °C.

Die erhaltenen Pellets können mittels pharmazeutisch üblicher Hilfsstoffe und in an sich bekannter Weise zu einer multipartikulären Arzneiform, insbesondere zu pellethaltigen Tabletten, Minitabletten, Kapseln, Sachets oder Trockensäften weiterverarbeitet werden, die so formuliert sind, daß die enthaltenen Pellets im pH-Bereich des Magens freigesetzt werden.

### Multipartikuläre Arzneiform

Die erfindungsgemäße Arzneiform, z. B. in Pelletform, kann vorteilhaft als Bestandteil einer multipartikulären Arzneiform verwendet werden. Bei der Verarbeitung in pharmaüblichen Produktionsvorgängen wie Verpressen, Abfüllen in Kapseln bzw. Sachets oder Mischen mit anderen Pelletpräparationen erweisen sich die verbesserten mechanischen Eigenschaften als besonders vorteilhaft. Die Vorteile treten insbesondere bei sehr dünnen Überzügen und/oder sehr hoher Wirkstoffbeladung hervor. Gerade bei Verpressen von Pellets zu Tabletten, wo besonders hohe mechanische Kräfte auftreten, erweist sich die erfindungsgemäße Arzneiform als wenig anfällig gegenüber Beschädigungen der Überzugsschichten. Die Folge ist eine hohe Prozesssicherheit und eine hohe Reproduzierbarkeit der Eigenschaften von Einheiten aus verschiedenen Produktionszyklen.

### Freisetzungscharakteristik

Die Wirkstofffreisetzungscharakteristik entspricht zwar nicht exakt derjenigen, er WO 01/68058 ist jedoch ähnlich. Die Abweichungen sind überraschend gering. Die Arzneiform eignet sich daher besonders gut für die Freisetzung von Wirkstoffen im Colon.

Im Freisetzungstest nach USP für zwei Stunden bei pH 1,2 und anschließendem Umpuffern auf pH 7,0 den enthaltenen Wirkstoff im Zeitraum bis 2,0 Stunden nach Testbeginn zu weniger als 5 % und zum Zeitpunkt acht Stunden nach Testbeginn zu 30 bis 80 %, insbesondere zu 40 bis 70 % freisetzt.

Der Freisetzungstest z. B. nach USP (nach USP XXIV, Methode B, modifizierter Test für "enteric coated products") ist dem Fachmann bekannt. Die Versuchsbedingungen sind insbesondere: Paddle-Methode, 100 Umdrehungen pro Minute, 37 °C; pH 1,2 mit 0,1 N HCl, pH 7,0 durch Zugabe von 0,2 M Phosphatpuffer und Einstellen mit 2 N NaOH. Siehe auch USP 27-NF22 Supplement 1, Methode "Delayed Release" Monographie <724> Drug Release.

Die zu verwendende mehrschichtige Arzneiform besteht im wesentlichen aus einem Kern mit einem Wirkstoff, einem inneren und einem äußeren Überzug. In üblicher Weise können pharmazeutisch gebräuchliche Hilfsstoffe enthalten sein, die aber für die Erfindung nicht kritisch sind.

### Pharmazeutische Wirkstoffe

Die im Sinne der Erfindung einsetzbaren pharmazeutischen Wirkstoffe sind dazu bestimmt, am oder im menschlichen oder tierischen Körper Anwendung zu finden, um
1. Krankheiten, Leiden, Körperschäden oder krankhafte Beschwerden zu heilen, zu lindern, zu verhüten oder zu erkennen.
2. die Beschaffenheit, den Zustand oder die Funktionen des Körpers oder seelische Zustände erkennen lassen.
3. vom menschlichen oder tierischen Körper erzeugte Wirkstoffe oder Körperflüssigkeiten zu ersetzen.
4. Krankheitserreger, Parasiten oder körperfremde Stoffe abzuwehren, zu beseitigen oder unschädlich zu machen oder
5. die Beschaffenheit, den Zustand oder die Funktionen des Körpers oder seelische Zustände zu beeinflussen.
Gebräuchliche Arzneistoffe sind Nachschlagewerken, wie z.B. der Roten Liste oder dem Merck Index zu entnehmen. Beispielhaft seien 5-Aminosalicylsäure, Corticosteroide (Budesonid), sowie Proteine (Insulin, Hormone, Antikörper) angeführt. Erfindungsgemäß können alle Wirkstoffe eingesetzt werden, die die gewünschte therapeutische Wirkung im Sinne der obigen Definition erfüllen und eine ausreichende Stabilität besitzen und deren Wirksamkeit gemäß der obigen Punkte über das Colon erreicht werden kann.
Wichtige Beispiele (Gruppen und Einzelsubstanzen) ohne Anspruch auf Vollständigkeit sind folgende:
Analgetika, Antibiotika, Antidiabetika, Antikörper
Chemotherapeutika, Corticoide/Corticosteroide
Entzündungshemmende Mittel, Enzympräparate
Hormone und deren Hemmstoffe, Nebenschilddrüsenhormone
Verdauungsfördernde Mittel, Vitamine, Zytostatika

Als Wirkstoffe sind insbesondere solche zu nennen, die im Darm, insbesondere kurz vor oder erst im Dickdarmbereich möglichst konstant freigesetzt werden sollen. Somit kann der pharmazeutische Wirkstoff ein Aminosalicylat, ein Sulfonamid oder ein Glucocorticoid sein, insbesondere 5-Aminosalicylsäure, Olsalazin, Sulfalazin, Prednison oder Budesonid.

### Beispiele für Wirkstoffe

Mesalazin
Sulfasalazin
Bethamethason-21-dihydrogenophosphat
Hydrocortison-21-acetat
Cromoglicinsäure
Dexamethason
Olsalazin-Na
Budesonid, Prednison
Bismunitrat, Karaya Gummi
Methylprednisolon-21-hydrogensuccinat
Myhrre, Kaffeekohle, Kamillenblüttenextrakt
10% Suspension von Humanplacenta

### Neuere Wirkstoffe, bzw. Wirkstoffe in der Entwicklung und Prüfung

(Literatur aus einschlägigen, dem Fachmann bekannten pharmazeutischen Datenbanken)
Balsalazid
Oral verabreichte Peptide (z.B. RDP 58)
Interleukin 6
Interleukin 12
Ilodecakin (Interleukin 10)
Nicotintartrat
5-ASA Konjugate (CPR 2015)
Monoclonaler Antikörper gegen Interleukin 12
Diethyldihydroxyhomospermin (DEHOHO)
Diethylhomospermin (DEHOP)
Cholecystokinin (CCK) Antagonist (CR 1795)
15 Aminosäure-Fragment eines 40 kd Peptids aus Magensaft (BPC 15)
Glucocorticoidanalogon (CBP 1011)
Natalizumab
Infliximab (REMICADE)
N-de-Acetyliertes Lysoglycosphingolipid (WILD 20)
Azelastine
Tranilast
Sudismase
Phosphorothioat Antisensoligonucleotid (ISIS 2302)
Tazofelone
Ropivacaine
5 Lipoxygenaseinhibitor (A 69412)
Sucralfat

Die Arzneiform kann einen pharmazeutischen Wirkstoff enthalten, der ein Enzym, ein Peptidhormon, ein immunmodulatorisches Protein, ein Antigen oder Antikörper ist.

Die Arzneiform kann als pharmazeutischen Wirkstoff ein Pankreatin, ein Insulin, ein Human Growth Hormon (hGH), Corbaplatin, Intron A, Calcitonin, Cromalyn, ein Interferon, ein Calcitonin, Granulocyte Colony Stimulating factor (G-CSF), ein Interleukin, Parathyroidhormone, Glucagon, Pro-Somatostatin, ein Somatostatin, Detirelix, Cetrorelix, Vasopressin, 1-Deaminocysteine-8-D-arginine-Vasopressin, Leuprolidacetat oder ein Antigen, das aus Gräsern oder anderen Pflanzen, wie z. B. Roggen, Weizen, Gerste, Hafer, Bermuda Gras, Zinnkraut, Ahorn, Ulme, Eiche, Platane, Pappel, Zeder, Zinnkraut, Disteln gewonnen wurde, enthalten.

### Pharmazeutisch übliche Hilfsstoffe

Von den pharmazeutisch übliche Hilfsstoffen im Sinne der Erfindung sind Porenbildner in Mengenanteilen ab 5 Gew.-%, bezogen auf den inneren Überzug ausgenommen.

Bei der Herstellung der mehrschichtigen Arzneiform können pharmazeutisch übliche Hilfsstoffe in üblicher Weise eingesetzt werden.

Trockenstellmittel (Antihaftmittel): Trockenstellmittel haben folgende Eigenschaften: sie verfügen über große spezifische Oberflächen, sind chemisch inert, sind gut rieselfähig und feinteilig. Aufgrund dieser Eigenschaften erniedrigen sie die Klebrigkeit von Polymeren, die als funktionelle Gruppen polare Comonomere enthalten.

### Beispiele für Trockenstellmittel sind:

Aluminiumoxid, Magnesiumoxid, Kaolin, Talkum, Glycerolmonostearat, Magnesiumstearat, Kieselsäure (Aerosile), Syloid, Bariumsulfat.

### Trennmittel

Beispiele für Trennmittel sind:
Ester von Fettsäuren oder Fettsäureamide , aliphatische, langkettige Carbonsäuren, Fettalkohole sowie deren Ester, Montan- oder Paraffinwachse und Metallseifen, insbesondere zu nennen sind Glycerolmonostearat, Stearylalkohol, Glycerolbehensäureester, Cetylalkohol, Palmitinsäure, Kanaubawachs, Bienenwachs etc.. Übliche Mengenanteile liegen im Bereich von 0,05 Gew-% bis 5, bevorzugt 0,1 bis 3 Gew.-% bezogen auf das Copolymere.

Weitere pharmazeutisch übliche Hilfsstoffe: Hier sind z. B, Stabilisatoren, Farbstoffe, Antioxidantien, Netzmittel, Pigmente, Glanzmittel etc. zu nennen. Sie dienen vor allem als Verarbeitungshilfsmittel und sollen ein sicheres und reproduzierbares Herstellungsverfahren sowie gute Langzeitlagerstabilität gewährleisten werden kann. Weitere pharmazeutisch übliche Hilfsstoffe können in Mengen von 0,001 Gew-% bis 30 Gew.-%, bevorzugt 0,1 bis 10 Gew.-% bezogen auf das Copolymere vorliegen.

Weichmacher: Als Weichmacher geeignete Stoffe haben in der Regel ein Molekulargewicht zwischen 100 und 20 000 und enthalten eine oder mehrere hydrophile Gruppen im Molekül, z. B. Hydroxyl- , Ester- oder Aminogruppen. Geeignet sind Citrate, Phthalate, Sebacate, Rizinusöl. Beispiele geeigneter Weichmacher sind Citronensäurealkylester, Glycerinester, Phthalsäurealkylester, Sebacinsäurealkylester, Sucroseester, Sorbitanester, Dibutylsebacat und Polyethylenglykole 4000 bis 20.000. Bevorzugte Weichmacher sind Tributylcitrat, Triethylcitrat, Acetyltriethylcitrat, Dibutylsebacat und Diethylsebacat. Die Einsatzmengen liegen zwischen 1 und 35, bevorzugt 2 bis 10 Gew.-%, bezogen auf das jeweilige Polymer oder Copolymer.

### Applikationsformen

Die beschriebene Arzneiform kann als überzogene Tablette, in Form einer Tablette aus verpreßten Pellets oder in Form von Pellets vorliegen, die in eine Kapsel, z. B. aus Gelatine, Stärke oder Cellulosederivaten, eingefüllt sind.

### BEISPIELE

### Prüfung mechanischer Eigenschaften von 1- und 2-schichtigen Filmüberzügen, die durch Ausgießen hergestellt wurden

EUDRAGIT^{®} RS: Copolymer aus 65 Gew.-% Methylmethacrylat, 30 Gew.-% Ethylacrylat und 5 Gew.-% 2-Trimethylammoniumethylmethacrylat-Chlorid.

EUDRAGIT^{®} RL: Copolymer aus 6 Gew.-% Methylmethacrylat, 30 Gew.-% Ethylacrylat und 10 Gew.-% 2-Trimethylammoniumethylmethacrylat-Chlorid.

EUDRAGIT^{®} NE: Copolymer aus 30 Gew.-% Ethylacrylat und 70 Gew.-% Methylmethacrylat.

EUDRAGIT^{®} FS: Copolymer aus 65 Gew.-% Methylacrylat, 25 Gew.-% Methylmethacrylat und 10 Gew.-% Methacrylsäure
1. Schicht = entspricht dem inneren Überzugsfilm in einer erfindungsgemäßen Arzneiform
2. Schicht = entspricht dem äußeren Überzugsfilm in einer erfindungsgemäße Arzneiform

### Herstellung der filmbildenden Formulierungen:

### EUDRAGIT^{®} FS 30 D Formulierung, 10 %-ig wässrig,

hergestellt aus einer 30 % igen EUDRAGIT FS 30 D Dispersion und 5 % (bezogen aufs Polymere) Triethylcitrat (TEC), mit deionisiertem Wasser wird die Dispersion auf 10 % verdünnt:
Im einem 400 ml Becherglas wurden TEC und Wasser eingewogen und bei 400 UpM auf dem Magnetrührer bis zur Auflösung des TEC gerührt bis zur klaren Lösung.
In einer 500 ml PE-Schraubflasche wird die über ein ca. 0,1 bis 0,2 mm Metallsieb filtrierte Menge von EUDRAGIT^{®} FS 30 D vorgelegt und unter Rührung mit dem Magnetrührer bei ca. 400 ± 100 UpM die wässrige TEC-Lösung dazugegeben.
Die Formulierung wird verschlossen bei Raumtemperatur mindestens
1 - 2 Stunden bei dieser Drehzahl gerührt.
Die 10 %-ige Dispersion wurde über Nacht im Kühlschrank bei 4 - 8°C gelagert werden und am nächsten Tag kurz vorm Ausgießen auf die Platte aufgerührt.

### EUDRAGIT^{®} RS 30 D / RL 30 D (1:1)Formulierung, 10 % ig wässrig,

hergestellt aus einer Mischung von jeweils 30 %-igen
EUDRAGIT^{®} RS 30 D / RL 30D (1:1) Dispersion und 20 % (bezogen aufs Polymere) Triethylcitrat, mit deionisiertem Wasser wird die Dispersion auf 10 % verdünnt:

Im einem 400 ml Becherglas wurden TEC und Wasser eingewogen und bei 500 Upm auf dem Magnetrührer bis zur Auflösung des TEC gerührt bis zur klaren Lösung.
In einer 500 ml PE-Schraubflasche wird die über ein ca. 0,1 bis 0,2 mm Metallsieb filtrierte Menge von EUDRAGIT^{®} RS 30 D / RL 30D (1:1) Dispersion vorgelegt und unter Rührung mit dem Magnetrührer bei ca. 400 ± 100 Upm die wässrige TEC-Lösung dazugegeben.
Die Formulierung wird verschlossen bei Raumtemperatur über Nacht bei dieser Drehzahl gerührt.

### EUDRAGIT^{®}NE 30D Formulierung, 10 % ig wässrig,

hergestellt aus einer 30 % igen EUDRAGIT^{®} NE 30 D Dispersion und verdünnt mit deionisiertem Wasser auf 10 % verdünnt:
In einer 500 ml PE-Schraubflasche wird die über ein ca. 0,1 bis 0,2 mm Metallsieb filtrierte Menge von EUDRAGIT^{®} NE 30 D vorgelegt und unter Rührung mit dem Magnetrührer bei ca. 400 ± 100 UpM das Wasser dazugegeben.
Die Formulierung wird verschlossen bei Raumtemperatur über Nacht bei dieser Drehzahl gerührt.

### Polyvinylacetat (Kollicoat^{®} SR 30 D) Formulierung, 10 % ig wässrig,

hergestellt aus einer 30 %-igen Polyvinylacetat Dispersion, 10 % (bezogen aufs Polymere) Propylenglykol und 3 % (bezogen aufs Polymere) Kollidon^{®} 25, mit deionisiertem Wasser wird die Dispersion auf 10 % verdünnt:
Im einem 400 ml Becherglas wurden Propylenglykol und Wasser eingewogen und bei 500 Upm auf dem Magnetrührer bis zur Auflösung des Propylenglykols gerührt.
Kollidon^{®} 25 wird dann bei Rührgeschwindigkeit von anfangs 300 bis später 990 Upm eingetragen und solange gerührt bis Kollidon 25 benetzt ist. Verklumpungen werden anschließend mit Hilfe eines Ultraturrax-Rührers ca. 15 min lang bei ca. 900 Upm Rührung aufgelöst. Die klare Lösung wird anschließend zur Luftblasenentweichung
5 min bei Raumtemperatur stehen gelassen.
In einer 500 ml PE-Schraubflasche wird die über ein ca. 0,1 bis 0,2 mm Metallsieb filtrierte Menge der Polyvinylacetat Dispersion vorgelegt und unter Rührung mit dem Magnetrührer bei ca. 400 ± 100 UpM die wässr. Propylenglykol - Kollidon^{®} 25 - Lösung dazugegeben.

Die Formulierung wird verschlossen bei Raumtemperatur über Nacht bei dieser Drehzahl gerührt.

### Filmeausgießen

### Vorbereitung der Ausgussplatten:

20 cm x20 cm große Glasplatten werden mit einem 2 cm Gewebeklebeband am Rand 3lagig abgeklebt, so dass man eine Umrandung von ca. 1 mm Höhe erhält und eine Ausguss-Innenfläche von ca. 256 cm².

Die ca. 256 cm² Ausguss-Innenfläche der Glasplatte wird dann mit einem Haftkleber einmal bepinselt und mit einem Warmluftfön angetrocknet.

Auf diese klebrige Fläche wird nun eine 20 cm x 20 cm große Aluminiumfolie von
Fa. TSCHELLIN mit der Mattseite nach oben beklebt, d.h. darauf glatt gewalzt oder mit einem Kuchenschaber bis in die Eckkanten glatt ausgestrichen. (Aluminiumfolie = 0,012 mm Dickenstärke, Seiten = glänzend/mattweich, Mattseite= lackkaschierte auf gefärbte biaxial gereckte Polypropylenfolie 0,03 mm).
Die über den Rand nichtklebende Aluminiumfolie wird nach oben gebogen, so dass man nun einen erhöhte Umrandungsfläche hat, die ein Überlaufen der Flüssigkeit verhindern kann.

Die so präparierten Ausguss-Glasplatten werden nun im Umlufttrockenschrank mit einer Libelle waagegerecht austariert ausgelegt.

### Herstellung 2-schichtiger Filme:

Alle hergestellten Formulierungen werden vor dem Ausgießen zur Filmherstellung jeweils über ein ca. 0,1 bis 0,2 mm Metallsieb filtriert.

Auf die präparierten und im Umlufttrockenschrank austarierten Ausguss-Glasplatten werden als 1. Grundschicht pro Platte jeweils 64 g einer über ein Metallsieb filtrierten
10 % igen EUDRAGIT^{®} FS 30 D Formulierung bei Raumtemperatur ausgegossen. Erst dann wird der Umlufttrockenschrank auf 50 °C geheizt und die Filme bei dieser Temperatur mit minimaler Ventilatorumdrehung und einer 30% geöffneten Luftklappe mindestens 3 Tage getrocknet.
Die nun klar aussehenden teilweise glatten FS 30 D - Filme werden nun im geöffneten Umlufttrockenschrank auf Raumtemperatur abgekühlt, bevor die 2. Filmschicht ausgegossen wird.
Pro EUDRAGIT^{®} - bzw. Konkurrenzprodukt- Probe werden 3 Ausguss-Glasplatten mit EUDRAGIT^{®} FS 30 D - Filmen als 1. Grundschicht verwendet. Auf diese EUDRAGIT^{®} FS 30 D - Filmgrundschicht wird nun jeweils 64 g einer über ein Metallsieb filtrierten 10 %-igen EUDRAGIT^{®} - bzw. anderen Probe ausgegossen.
Auch hier wird erst dann nach dem Ausgießen der Formulierungen der Umlufttrockenschrank auf 50 °C geheizt und die Filme bei dieser Temperatur mit minimaler Ventilatorumdrehung und einer 30% geöffneten Luftklappe mindestens 3 bis 5 Tage getrocknet bis die Filme ein klares Aussehen erhalten, Ausnahme: 2-schichtiger Film mit Polyvinylacetat (Kollicoat^{®} SR 30 D) zeigt eine leicht gelblich milchige Trübung (5 Tage-Trocknung) und mit Ethylcellulose (Aquacoat^{®} ECD-30) eine leicht rissige Trübung mit Haftungsproblemen zum Unterfilm (3 Tage-Trocknung).

Die nun erhaltenen 2-schichtigen Filme werden auf Raumtemperatur abgekühlt, vorsichtig von der Aluminiumfolie gelöst und jeder für sich in Filterpapier geformte Taschen aufbewahrt, die wiederum in einem PE-Beutel eingeschweißt werden.

### Herstellung 1-schichtiger Filme:

Alle hergestellten Formulierungen werden vor dem Ausgießen zur Filmherstellung jeweils über ein ca. 0,1 bis 0,2 mm Metallsieb filtriert.

Auf die präparierten und im Umlufttrockenschrank austarierten Ausguss-Glasplatten werden pro Probe je 2 Platten mit jeweils 100 g einer über ein Metallsieb filtrierten
10 % igen EUDRAGIT^{®} - bzw. Konkurrenzprodukt -Formulierung bzw. 67 g einer über ein Metallsieb filtrierten 15 % igen Formulierung (z.B. kolloidale Lösung von Formulierung) bei Raumtemperatur ausgegossen.
Erst dann wird der Umlufttrockenschrank auf 50 °C geheizt und die Filme bei dieser Temperatur mit minimaler Ventilatorumdrehung und einer 30% geöffneten Luftklappe mindestens 3 Tage getrocknet. Nach dieser Zeit zeigen die Filme von der Formulierung EUDRAGIT^{®} FS 30 D und EUDRAGIT^{®} NE 30 D ein klares Aussehen, Aquacoat^{®} ECD-30 ergibt einen sehr spröden Film, der schon bei der Handhabung zerbricht und somit nicht mehr bestimmbar ist. Filmformulierungen von Kollicoat^{®} SR 30 D und EUDRAGIT^{®} RS 30 D / RL 30 D (1:1) können nach 3 Tagen nochmals bei 60 °C über Nacht zur Entfernung der Restfeuchte temperiert werden. Dabei muss auf Blasenbildung geachtet werden. Das Aussehen bei EUDRAGIT^{®} RS 30 D / RL 30 D (1:1) ist dann klar bis minimal trüb und bei Kollicoat^{®} SR 30 D gelblich trüb.
Die nun erhaltenen 1-schichtigen Filme werden auf Raumtemperatur abgekühlt, vorsichtig von der Aluminiumfolie gelöst und jeder für sich in Filterpapier geformte Taschen aufbewahrt, die wiederum in einen PE-Beutel eingeschweißt werden.

### Zugversuch:

| | |
|---|---|
| Vorschrift: | ISO 527-2 /1BA / 20 |
| Prüfklima: | 23°C / 50 % rel. F. |
| Spannzeug: | Luft |
| Maschine: | Genauigkeitsklasse 1 % |
| Wegaufnehmer: | Traverse |
| Einspannlänge: | 57,5 mm |
| Konditionierung: | 16 Std. Normklima (23°C / 50 % rel. F.) |
| Messlänge: | 57,5 mm |
| Vorlast: | 0,05 MPa |

### Beispiel 1-10

| Bsp. | | Polymere | Zugfestigkeit | Nom. Bruchdehnung |
|---|---|---|---|---|
| | | | [Mpa] | [%] |
| 1 | nicht erfindungsgemäß | 1 Schicht EUDRAGIT^{®} FS | 10,1 | 187 |
| 2 | nicht erfindungsgemäß | 1 Schicht EUDRAGIT^{®} RL/RS (1:1) | 1,5 | 257 |
| 4 | nicht erfindungsgemäß | 1 Schicht EUDRAGIT^{®} NE | 4,1 | 819 |
| 5 | nicht erfindungsgemäß | 1 Schicht Polyvinylacetat | 10,0 | 450 |
| 6 | nicht erfindungsgemäß (gemäß WO 01/68058) | 1. Schicht: EUDRAGIT^{®} RL/RS (1:1) | 5,4 | 174 |
| | | 2. Schicht EUDRAGIT^{®} FS | | |
| 7 | nicht erfindungsgemäß | 1. Schicht: Ethylcellulose | Nicht bestimmbar, weil sich die Schichten schon bei der Probenvorbereitung trennen. | |
| | | 2. Schicht EUDRAGIT^{®} FS | | |
| 9 | erfindungsgemäß | 1. Schicht EUDRAGIT^{®} NE | 7,0 | 174 |
| | | 2. Schicht EUDRAGIT^{®} FS | | |
| 10 | erfindungsgemäß | 1. Schicht Polyvinylacetat | 8,0 | 288 |
| | | 2. Schicht EUDRAGIT^{®} FS | | |

Man erkennt aus den Messwerten, dass alle zweischichtigen Polymersysteme, die an sich gute Festigkeit einer EUDRAGIT^{®} FS- Schicht reduzieren. Besonders stark ist dieser Effekt bei der Kombination gemäß WO 01/68058, Beispiel 6.

Rasterelekronenmikroskopische Aufnahmen von Querschnitten der Filme zeigen für alle erfindungsgemäßen, doppelschichtigen Filme homogene, gleichmäßige Schichten mit guter Haftung an der Grenzfläche.

## Patentansprüche

1. Mehrschichtige Arzneiform, enthaltend
a) einen Kern mit einem pharmazeutischen Wirkstoff
b) einen inneren Überzug, der zu 50 bis 95 Gew.-% aus einem (Co)polymeren besteht, das sich zu 95 bis 100 Gew.-% aus radikalisch polymerisierten vinylischen Monomeren mit neutralen Seitengruppen und 0 bis 5 Gew.-% Monomeren mit anionischen Seitengruppen zusammensetzt,
c) einen äußeren Überzug aus einem Copolymeren, das sich aus 75 bis 95 Gew.-% radikalisch polymerisierten C₁- bis C₄-Alkylestern der Acryl- oder der Methacrylsäure und 5 bis 25 Gew.-% (Meth)acrylat-Monomeren mit einer anionischen Gruppe im Alkylrest zusammensetzt, wobei 5 bis 30 Gew.-% an pharmazeutisch üblichen Hilfsstoffen enthalten sind,
**dadurch gekennzeichnet, daß**
der innere Überzug 5 bis 50 Gew.-% an pharmazeutisch üblichen Hilfsstoffen enthält, die keine Porenbildner sind und Porenbildner nur in Mengen von weniger als 5 Gew.-% enthalten sind.

2. Arzneiform nach Anspruch 1, **dadurch gekennzeichnet, daß** der innere Überzug ein (Co)polymer enthält, das sich zu 95 bis 100 Gew.-% aus radikalisch polymerisierten C₁- bis C₄-Alkylestern der Acryl- oder der Methacrylsäure und gegebenenfalls 0 bis 5 Gew.-% Acryl- oder Methacrylsäure zusammensetzt.

3. Arzneiform nach Anspruch 1, **dadurch gekennzeichnet, daß** der innere Überzug ein (Co)polymer enthält, das ein Polyvinylacetat ist.

4. Arzneiform nach einem oder mehreren der Ansprüche 1 bis 3, **dadurch gekennzeichnet, daß** die pharmazeutisch üblichen Hilfsstoffe, die in inneren Überzug enthalten sein können, aus den Stoffklassen der Weichmacher, Stabilisatoren, Farbstoffe, Antioxidantien, Netzmittel, Pigmente, Glanzmittel, Trennmittel, Trockenstellmittel ausgewählt sind, wobei Porenbildner, insbesondere wasserunlösliche Porenbildner wie Kaolin, Calciumcarbonat, Calciumhydrogenphosphat, Magnesiumoxid, mikrokristalline Cellulose, Titandioxid oder Eisenoxid, und insbesondere wasserlösliche Porenbildner wie Povidone K30, Polyvinylalkohol, Cellulosederivate, wie Hydroxypropylcellulose, Hydroxypropylmethylcellulose (HPMC), Methylcellulose oder Natriumcarboxymethylcellulose, Saccharose, Xylit, Sorbit, Mannit, Maltose, Xylose, Glucose, Kaliumchlorid, Natriumchlorid, Polysorbat 80, Poylethylenglykol oder Natriumcitrat nicht oder nur in Mengen von weniger als 5 Gew.-% enthalten sind.

5. Arzneiform nach einem oder mehreren der Ansprüche 1 bis 4, **dadurch gekennzeichnet, daß** das Gesamtgewicht des inneren Überzugs 2 bis 50 Gew.-% bezogen auf das Gesamtgewicht des Kerns ausmacht.

6. Arzneiform nach einem oder mehreren der Ansprüche 1 bis 5, **dadurch gekennzeichnet, daß** das Gesamtgewicht des äußeren Überzugs 5 bis 50 Gew.-% bezogen auf das Gesamtgewicht des Kerns und des inneren Überzugs ausmacht.

7. Arzneiform nach einem oder mehreren der Ansprüche 1 bis 6, **dadurch gekennzeichnet, daß** der enthaltene pharmazeutische Wirkstoff ein Aminosalicylat, ein Sulfonamid oder ein Glucocorticoid ist.

8. Arzneiform nach Anspruch 7, **dadurch gekennzeichnet, daß** der pharmazeutische Wirkstoff 5-Aminosalicylsäure, Olsalazin, Sulfalazin, Prednison oder Budesonid ist.

9. Arzneiform nach einem oder mehreren der Ansprüche 1 bis 6, **dadurch gekennzeichnet, daß** der pharmazeutische Wirkstoff ein Enzym, ein Peptidhormon, ein immunmodulatorisches Protein, ein Antigen oder Antikörper ist.

10. Arzneiform nach Anspruch 5, **dadurch gekennzeichnet, daß** der pharmazeutische Wirkstoff ein Pankreatin, ein Insulin, ein Human Growth Hormon (hGH), Corbaplatin, Intron A, Calcitonin, Cromalyn, ein Interferon, ein Calcitonin, Granulocyte Colony Stimulating factor (G-CSF), ein Interleukin, Parathyroidhormone, Glucagon, Pro-Somatostatin, ein Somatostatin, Detirelix, Cetrorelix, Vasopressin, 1-Deaminocysteine-8-D-arginine-Vasopressin, Leuprolidacetat oder ein Antigen, das aus Gräsern oder anderen Pflanzen, wie z. B. Roggen, Weizen, Gerste, Hafer, Bermuda Gras, Zinnkraut, Ahorn, Ulme, Eiche, Platane, Pappel, Zeder, Zinnkraut, Disteln gewonnen wurde.

11. Verfahren zur Herstellung von einer Arzneiform nach einem oder mehreren der Ansprüche 1 bis 10, **gekennzeichnet durch** die Schritte
a) Erzeugen eines Kerns mit einem pharmazeutischen mittels Sprühauftrag auf einen Neutral-Kern (Non-Pareilles) oder **durch** Rotagglomeration, Ausfällen, Sprühverfahren oder Extrusion und Spheronisation ohne einen Neutral-Kern herstellt und anschließend
b) Auftragen des inneren Überzugs, mittels Sprühauftrag, so daß wirkstoffhaltige, umhüllte Pellets erhalten werden,
c) Auftragen des äußeren Überzugs, mittels Sprühauftrag, so daß wirkstoffhaltige, zweifach umhüllte Pellets erhalten werden,
d) optional eine abschließende Curing-Behandlung zur Stabilisierung des Freigabeprofils, z. B. **durch** trockenes Lagern für 2 Stunden bei 40 °C.

12. Verfahren nach Anspruch 11, **dadurch gekennzeichnet, daß** man die erhaltenen Pellets mittels pharmazeutisch üblicher Hilfsstoffe und in an sich bekannter Weise zu einer multipartikulären Arzneiform, insbesondere zu pellethaltigen Tabletten, Minitabletten, Kapseln, Sachets oder Trockensäften verarbeitet, die so formuliert sind, daß die enthaltenen Pellets im pH-Bereich des Magens freigesetzt werden.

13. Verwendung einer Arzneiform nach einem oder mehreren der Ansprüche Ansprüche 1 bis 10, als Bestandteil einer multipartikulären Arzneiform.

14. Verwendung nach Anspruch 13 als Bestandteil von verpreßten Tablette, Kapseln, Sachets oder Trockensäften.

## Claims

1. A multilayer pharmaceutical form comprising
a) a core with an active pharmaceutical ingredient
b) an inner coating which consists of 50 to 95% by weight of a (co)polymer which is composed of 95 to 100% by weight of free-radical-polymerized vinylic monomers having neutral side groups and 0 to 5% by weight of monomers having anionic side groups,
c) an outer coating of a copolymer which is composed of 75 to 95% by weight of free-radical-polymerized C₁- to C₄-alkyl esters of acrylic or of methacrylic acid and 5 to 25% by weight of (meth)acrylate monomers having an anionic group in the alkyl radical, where 5 to 30% by weight of pharmaceutically usual excipients are present,
**characterized in that**
the inner coating comprises 5 to 50% by weight of pharmaceutically usual excipients which are not pore formers, and pore formers are present only in amounts of less than 5% by weight.

2. The pharmaceutical form as claimed in claim 1, **characterized in that** the inner coating comprises a (co)polymer which is composed of 95 to 100% by weight of free-radical-polymerized C₁- to C₄-alkyl esters of acrylic or of methacrylic acid and where appropriate 0 to 5% by weight of acrylic or methacrylic acid.

3. The pharmaceutical form as claimed in claim 1, **characterized in that** the inner coating comprises a (co)polymer which is a polyvinyl acetate.

4. The pharmaceutical form as claimed in one or more of claims 1 to 3, **characterized in that** the pharmaceutically usual excipients which may be present in the inner coating are selected from the substance classes of plasticizers, stabilizers, colorants, antioxidants, wetting agents, pigments, gloss agents, mold release agents, antitack agents, with the content of pore formers, in particular water-insoluble pore formers such as kaolin, calcium carbonate, calcium hydrogen phosphate, magnesium oxide, microcrystalline cellulose, titanium dioxide or iron oxide, and especially water-soluble pore formers such as povidone K30, polyvinyl alcohol, cellulose derivatives such as hydroxypropylcellulose, hydroxypropylmethylcellulose (HPMC), methylcellulose or sodium carboxymethylcellulose, sucrose, xylitol, sorbitol, mannitol, maltose, xylose, glucose, potassium chloride, sodium chloride, polysorbate 80, polyethylene glycol or sodium citrate, being zero or only amounts of less than 5% by weight.

5. The pharmaceutical form as claimed in one or more of claims 1 to 4, **characterized in that** the total weight of the inner coating amounts to 2 to 50% by weight based on the total weight of the core.

6. The pharmaceutical form as claimed in one or more of claims 1 to 5, **characterized in that** the total weight of the outer coating amounts to 5 to 50% by weight based on the total weight of the core and of the inner coating.

7. The pharmaceutical form as claimed in one or more of claims 1 to 6, **characterized in that** the contained active pharmaceutical ingredient is an aminosalicylate, a sulfonamide or a glucocorticoid.

8. The pharmaceutical form as claimed in claim 7, **characterized in that** the active pharmaceutical ingredient is 5-aminosalicylic acid, olsalazine, sulfasalazine, prednisone or budesonide.

9. The pharmaceutical form as claimed in one or more of claims 1 to 6, **characterized in that** the active pharmaceutical ingredient is an enzyme, a peptide hormone, an immunomodulatory protein, an antigen or antibody.

10. The pharmaceutical form as claimed in claim 5, **characterized in that** the active pharmaceutical ingredient is a pancreatin, an insulin, a human growth hormone (hGH), carboplatin, intron A, calcitonin, cromalyn, an interferon, granulocyte colony stimulating factor (G-CSF), an interleukin, parathyroid hormones, glucagon, pro-somatostatin, a somatostatin, detirelix, cetrorelix, vasopressin, 1-deaminocysteine-8-D-arginine-vasopressin, leuprolide acetate or an antigen which has been isolated from grasses or other plants such as, for example, rye, wheat, barley, oats, bermuda grass, horsetail, sycamore, elm, oak, plane tree, poplar, cedar, thistles.

11. A process for producing a pharmaceutical form as claimed in one or more of claims 1 to 10, **characterized by** the steps
a) production of a core having an active pharmaceutical ingredient by means of spray application to a neutral core (nonpareilles) or by rotagglomeration, precipitation, spray processes or extrusion and spheronization without a neutral core and subsequently
b) application of the inner coating by spray application so that active ingredient-containing, coated pellets are obtained,
c) application of the outer coating by spray application so that active ingredient-containing, doubly coated pellets are obtained,
d) optionally a final curing treatment to stabilize the release profile, e.g. by storing in the dry at 40°C for 2 hours.

12. The process as claimed in claim 11, **characterized in that** the resulting pellets are processed with the aid of pharmaceutically usual excipients and in a manner known per se to a multiparticulate pharmaceutical form, in particular to pellet-containing tablets, minitablets, capsules, sachets or reconstitutable powders which are formulated so that the contained pellets are released in the pH range of the stomach.

13. The use of a pharmaceutical form as claimed in one or more of claims 1 to 10 as constituent of a multiparticulate pharmaceutical form.

14. The use as claimed in claim 13 as constituent of compressed tablet, capsules, sachets or reconstitutable powders.

## Revendications

1. Forme médicamenteuse à plusieurs couches, contenant
a) un noyau avec une substance active pharmaceutique
b) un revêtement interne, qui est constitué, à raison de 50 à 95 % en poids, par un (co)polymère, qui est composé, à raison de 95 à 100 % en poids, de monomères vinyliques polymérisés par voie radicalaire avec des groupes latéraux neutres et à raison de 0 à 5 % en poids de monomères avec des groupes latéraux anioniques,
c) un revêtement externe en un copolymère qui est composé de 75 à 95 % en poids d'esters C₁-C₄-alkyliques de l'acide acrylique ou méthacrylique, polymérisés par voie radicalaire, et de 5 à 25 % en poids de monomères de (méth)acrylate renfermant un groupe anionique dans le radical alkyle, contenant 5 à 30 % en poids d'adjuvants pharmaceutiquement usuels,
**caractérisée en ce que** le revêtement interne contient 5 à 50 % en poids d'adjuvants pharmaceutiquement usuels qui ne sont pas des agents de formation de pores et les agents de formation de pores ne sont contenus qu'en des quantités inférieures à 5 % en poids.

2. Forme médicamenteuse selon la revendication 1, **caractérisée en ce que** le revêtement interne contient un (co)polymère qui est composé à raison de 95 à 100 % en poids d'esters C₁-C₄-alkyliques de l'acide acrylique ou méthacrylique polymérisés par voie radicalaire et le cas échéant à raison de 0 à 5 % en poids d'acide acrylique ou méthacrylique.

3. Forme médicamenteuse selon la revendication 1, **caractérisée en ce que** le revêtement interne contient un (co)polymère qui est un poly(acétate de vinyle).

4. Forme médicamenteuse selon l'une ou plusieurs des revendications 1 à 3, **caractérisée en ce que** les adjuvants pharmaceutiquement usuels qui peuvent être contenus dans le revêtement interne sont choisis parmi les classes des plastifiants, des stabilisateurs, des colorants, des antioxydants, des agents mouillants, des pigments, des agents brillants, des agents de démoulage, des agents antiadhésifs, où les agents de formation de pores, en particulier les agents de formation de pores insolubles dans l'eau tels que le kaolin, le carbonate de calcium, l'hydrogénocarbonate de calcium, l'oxyde de magnésium, la cellulose microcristalline, le dioxyde de titane ou l'oxyde de fer et en particulier les agents de formation de pores solubles dans l'eau, tels que la Povidone K30, le poly(alcool vinylique), les dérivés de cellulose, tels que l'hydroxypropylcellulose, l'hydroxypropylméthylcellulose (HPMC), la méthylcellulose ou la carboxyméthylcellulose sodique, le saccharose, le xylitol, le sorbitol, le mannitol, le maltose, le xylose, le glucose, le chlorure de potassium, le chlorure de sodium, le Polysorbate 80, le polyéthylèneglycol ou le citrate de sodium ne sont pas contenus ou seulement en des quantités de moins de 5 % en poids.

5. Forme médicamenteuse selon l'une ou plusieurs des revendications 1 à 4, **caractérisée en ce que** le poids total du revêtement interne représente 2 à 50 % en poids par rapport au poids total du noyau.

6. Forme médicamenteuse selon l'une ou plusieurs des revendications 1 à 5, **caractérisée en ce que** le poids total du revêtement externe représente 5 à 50 % en poids par rapport au poids total du noyau et du revêtement interne.

7. Forme médicamenteuse selon l'une ou plusieurs des revendications 1 à 6, **caractérisée en ce que** la substance active pharmaceutique est contenue un aminosalicylate, un sulfonamide ou un glucocorticoïde.

8. Forme médicamenteuse selon la revendication 7, **caractérisée en ce que** la substance active pharmaceutique est l'acide 5-aminosalicylique, l'olsalazine, la sulfasalazine, le prednisone ou le budésonide.

9. Forme médicamenteuse selon l'une ou plusieurs des revendications 1 à 6, **caractérisée en ce que** la substance active pharmaceutique est une enzyme, une hormone peptidique, une protéine d'immunomodulation, un antigène ou un anticorps.

10. Forme médicamenteuse selon la revendication 5, **caractérisée en ce que** la substance active pharmaceutique est une pancréatine, une insuline, une hormone de croissance humaine (hGH), le carboplatine, l'Intron A, la calcitonine, la cromalyne, un interféron, un facteur de stimulation des colonies de granulocytes (G-CSF), une interleukine, une hormone parathyroïdienne, le glucagon, la pro-somatostatine, une somatostatine, le detirelix, le cetrorelix, la vasopressine, la 1-désaminocystéine-8-D-arginine-vasopressine, l'acétate de leuprolide ou un antigène, qui est produit à partir de graminées ou d'autres plantes, telles que par exemple le seigle, le blé, l'orge, l'avoine, le cynodon dactyle (chiendent cultivé), la prèle, l'érable, l'orme, le chêne, le platane, le peuplier, le cèdre, les chardons.

11. Procédé pour la préparation d'une forme médicamenteuse selon l'une ou plusieurs des revendications 1 à 10, **caractérisé par** les étapes
a) de production d'un noyau avec une substance active pharmaceutique, par application par pulvérisation sur un noyau neutre (Nonpareilles) ou par agglomération rotative, précipitation, des procédés de pulvérisation ou extrusion et sphéronisation sans noyau neutre, puis
b) d'application d'un revêtement interne, par application par pulvérisation, de manière à obtenir des pellets enrobés contenant une substance active,
c) d'application d'un revêtement externe, par application par pulvérisation, de manière à obtenir des pellets doublement enrobés contenant une substance active,
d) éventuellement de traitement terminal de durcissement pour la stabilisation du profil de libération, par exemple par un entreposage à sec pendant 2 heures à 40°C.

12. Procédé selon la revendication 11, **caractérisé en ce qu'**on transforme les pellets obtenus au moyen d'adjuvants pharmaceutiquement usuels et de manière connue en soi en une forme médicamenteuse multiparticulaire, en particulier en comprimés, minicomprimés, capsules, sachets ou poudres reconstituables, contenant des pellets, qui sont formulés de manière telle que les pellets contenus sont libérés dans la plage de pH de l'estomac.

13. Utilisation d'une forme médicamenteuse selon l'une ou plusieurs des revendications 1 à 10, comme constituant d'une forme médicamenteuse multiparticulaire.

14. Utilisation selon la revendication 13 comme constituant de comprimés pressés, de capsules, de sachets ou de poudres reconstituables.
